# NEW EUROPEAN PATENT SPECIFICATION

(11) **EP 0 751 128 B2**
(45) Date of publication and mention of the opposition decision: **31.08.2005**
(45) Mention of the grant of the patent: 28.07.1999
(21) Application number: 96304797.2
(22) Date of filing: 28.06.1996
(51) Int. Cl.: C07D 217/26, C07D 498/04

(54) **Process for producing optically active amides**
Verfahren zur Herstellung von optisch aktiven Amiden
Procédé pour la préparation d'amides optiquement actifs

(30) Priority: 30.06.1995 JP 16653695
(43) Date of publication of application: 02.01.1997
(73) Proprietor: Ajinomoto Co., Inc., Tokyo (JP)
(72) Inventor: Sato, Takahiro, c/o Central Research Laboratories, Kawasaki-ku, Kawasaki-shi, Kanagawa-ken (JP); Izawa, Kunisuke, c/o Central Research Laboratories, Kawasaki-ku, Kawasaki-shi, Kanagawa-ken (JP)
(74) Representative: Nicholls, Kathryn Margaret

(56) References cited:
- EP-A- 0 162 877
- WO-A-97/30976
- US-A- 5 256 783
- CHIMIKA CHRONIKA - NEW SERIES, vol. 18, 1989, pages 3-17, XP000602305 E.C. WEIR ET AL.: "Tricyclic Hydantoins and Thiohydantoins of Phenylalanine"

## Description

The present invention relates to a process for producing optically active amides. It is particularly concerned with decahydro(4aS, 8aS)isoquinoline-3(S)-carboxamides:

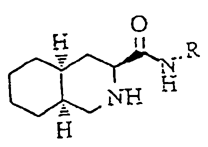

Such compounds are useful as intermediates in the production of Saquinavir (EP 432694) which is expected to find use as an anti-AIDS agent owing to its excellent HIV protease inhibition activity.

A method of producing decahydro (4aS, 8aS) isoquinoline-3(S)-carboxamides, is disclosed in US-A-5,256,783. The method described in this patent comprises five steps: N-protecting L-phenylalanine with benzyl chloroformate; converting the carboxy-group to N-tert-butylamido (84.3%); reacting the resulting amide with formaldehyde in the presence of an acid catalyst to form a tetrahydroisoquinoline compound (66%); deprotecting the ring nitrogen atom by catalytic reduction using Pd (79%); and finally hydrogenating this to N-tert-butyl decahydro(4aS, 8aS)isoquinoline 3(S) carboxamide using an Rh catalyst (59%). However, this method is problematic in that it requires many steps, some of low yield; in that expensive Rh is used; and in that the reaction has to be strictly controlled to maintain optical purity. Accordingly, there is scope for a better method.

It is known to use a non-homogenous metal catalyst such as expensive Rh, Pt or the like for nuclear reduction of an aromatic ring having a functional group. It is also known that inexpensive Ru can be used for nuclear reduction of an aromatic ring. However, this is nuclear reduction of an aromatic ring of a compound without functional groups, such as toluene. The influence on optical activity or sterically selective reduction is not known.

It is known to produce tetrahydroisoquinoline-3-carboxamide by a method in which tetrahydroisoquinoline-3-carboxylic acid is protected with a benzyloxycarbonyl group (66.5%), the resulting compound is converted into NCA: 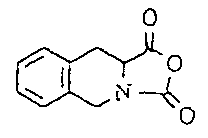 with phosphorus pentachloride (69%), and NCA is reacted with an amine to form an amide (57%) (Chimika Chronika, New Series, 18, 3, 1989). However, the yield in each step is low, and undesirable by-products and industrial waste are formed in large amounts. Accordingly, this method is not appropriate industrially. Furthermore since this method is conducted using a racemic compound, it is unclear whether or not optical activity would be maintained.

The present inventors have assiduously conducted investigations to provide a selective, industrially useful process for producing decahydro(4aS,8aS)isoquinoline-3(S)-carboxamides. They have found that tetrahydroisoquinolinecarboxamides can be formed in high yield from tetrahydroisoquinoline-3(S)-carboxylic acid, which is available by an established industrial process. The acid is reacted with phosgene, phosgene dimer or triphosgene to form tetrahydroisoquinolinecarboxylic acid N-carboxy anhydride (NCA). This can be done in high yield without impairing the optical activity. The NCA is then ring-opened by reaction with an amine to obtain an amide.

Furthermore the nuclear reduction of the aromatic ring of tetrahydroisoquinolinecarboxamide has been studied, and it has been consequently found that the aromatic ring can be reduced stereoselectively, maintaining the optical activity, with inexpensive Ru. Furthermore the yield given with Ru can be higher than that with Rh.

Thus the present invention provides a process for producing a tetrahydroisoquinoline-3(S)-carboxamide derivative represented by formula (3), which comprises reacting tetrahydroisoquinoline-3 (S) -carboxylic acid represented by formula (1) with at least one of phosgene, phosgene dimer and triphosgene to form the N-carboxy anhydride (NCA) represented by formula (2), and then reacting NCA (without its being isolated or purified) with an amine RNH₂ (where R is t-butyl); thereby to produce the optically active amide. The invention further provides a process for producing a decahydro (4aS,8aS)isoquinoline-3(S)-carboxamide derivative represented by formula (4), which further comprises reducing the compound of formula (3) in the presence of a metal catalyst. 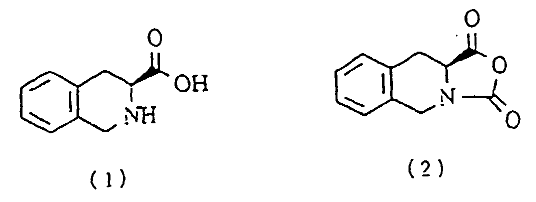 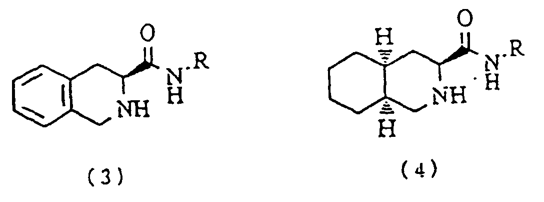 (R is a t-butyl group).

It is known that tetrahydroisoquinolinecarboxylic acid, as used in the present invention, is easily obtained by reacting phenylalanine with formaldehyde in the presence of an acid catalyst [Pictet Spengler reaction; Chem. Pharm. Bull., 31, 312, 1983 and Japanese Laid-Open Patent Application (Kokai) No. 157,466/1994]. It is industrially mass-produced as an intermediate of Quinapril, an ACE inhibitor.

The reaction of tetrahydroisoquinoline-3(S)-carboxylic acid (1) with at least one of phosgene, phosgene dimer and triphosgene (hereinafter abbreviated as "phosgene or the like") to form N-carboxy anhydride (2) (hereinafter abbreviated as "NCA") may be carried out by dissolving or suspending the acid (1) in an organic solvent, and adding phosgene or the like to the solution to form NCA.

It is also possible to dissolve phosgene or the like in the organic solvent and add the acid (1) to the solution. Phosgene or the like used in the reaction may be a monomer (phosgene gas), a dimer (phosgene dimer) or a trimer (triphosgene). It is generally used in an amount of from 1 to 10 equivalents, preferably from 1.1 to 1.3 equivalents based on the starting material (1). The organic solvent used is not particularly limited provided it does not react with phosgene. Tetrahydrofuran (THF) is preferable since it does not react with an amine in the next step, and is also a solvent for tetrahydroisoquinoline-3(S)-carboxamide. The reaction is generally conducted at a temperature of from 0 to 100°C, preferably from 40 to 70°C. The reaction time is generally between 0.1 and 36 hours, usually between 2 and 5 hours.

NCA(2) obtained by the reaction can be isolated as crystals by cooling the reaction solution or adding an inert solvent in which NCA is less soluble, e.g. hexane, heptane, toluene or dichloromethane.

The optically active (3S) form of NCA(2) is a novel compound from which the tetrahydroisoquinoline-3(S)-carboxamide derivative (3) can easily be formed with retention of optical activity.

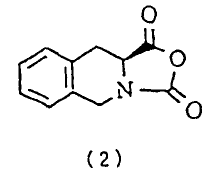

NCA (2) is used for the subsequent amidation step as it is, without being isolated or purified. It is preferable that the NCA solution be added to the t-butylamine. The solvent for the NCA is not particularly limited provided it does not react with NCA or the amine. Tetrahydrofuran (THF) and dichloromethane are preferable in that these may also be used in the preceding reaction. The amine may be dissolved in a solvent or used as it is. The amount of the amine is generally from 1 to 50 equivalents, preferably 2 to 5 equivalents, based on NCA. The reaction proceeds approximately quantitatively at a reaction temperature of from -50 to 70°C, preferably from 0 to 20°C. The reaction time is generally between 0.01 and 24 hours, usually between 1 and 5 hours.

The tetrahydroisoquinoline-3(S)-carboxamide derivative (3) in which R is a tert-butyl group as obtained by the above-mentioned reaction coincided with the product which was separately formed by the process described in U. S. Patent No. 5,256,783 with respect to all analytical values.

Assuming a yield of 81% for the preparation of tetrahydroisoquinolinecarboxylic acid from phenylalanine as described in Japanese Laid-Open Patent Application (Kokai) No. 157,466/1994, then the present invention enables the conversion of phenylalanine into N-tert-butyltetrahydroisoquinolinecarboxamide with an overall yield of 63%. Compared with the yield of 44% in U.S. Patent No. 5,256,783, this is an improvement of more than 40%.

The step of reducing the tetrahydroisoquinoline-3(S)-carboxamide derivative (3) into the decahydroisoquinoline-3(S)-carboxamide derivative (4) can be conducted by dissolving the tetrahydroisoquinoline-3(S)-carboxamide in a solvent, then adding a metal catalyst thereto, and conducting the reaction in the presence of hydrogen. Examples of the metal catalyst used in the reaction include Rh, Pt and Ru. Especially when using Ru, the reduction proceeds stereoselectively without causing racemization, and the decahydro-(4aS,8aS)-isoquinoline-3 (S) -carboxamide derivative (4) can be formed in good yield. As forms of Ru catalyst, Ru/C and Ru/alumina are available.

As the solvent used in the reaction, a solvent which is free from aromatic rings and which is unreactive with the substrate should be used. Such solvents includes alcohols, esters, acetic acid and water. 2-Propanol is preferable with respect to the vapor pressure in the reaction and the treatment after the reaction. The hydrogen pressure may be between 5 and 200 atm, preferably between 10 and 50 atm on grounds of economics and reactivity. The reaction temperature may be between 20 and 200°C, preferably between 80 and 120°C with regard to the optical purity.

After the completion of the reaction, the catalyst may be separated by filtration, and the residue is concentrated. The concentrate can easily be purified by crystallisation using an appropriate solvent, for example, a hydrocarbon solvent such as hexane or heptane. Or it can be crystallized as a salt, e.g. with hydrochloric acid or an organic acid. The thus-obtained crystals do not contain stereoisomers and can easily be isolated and purified. Therefore, an industrially useful process can be provided.

### Examples

### Synthesis Example 1

One gram (5.64 mmols) of tetrahydroisoquinoline-3(S)-carboxylic acid (made by Aldrich) and a solution of 0.67 g (2.26 mmols) of triphosgene in 10 ml of tetrahydrofuran were heated and stirred at 60°C for 3 hours. After the completion of the reaction,the solvent was distilled off, and the residue was dissolved in 5ml of tetrahydrofuran and 10ml of dichloromethane. The resulting slurry was added dropwise to a solution of 2.97 ml (28.2 mmols) of tert-butylamine in 10 ml of tetrahydrofuran. The mixture was stirred overnight at room temperature. The reaction mixture was acidified with 30 ml of 1-N hydrochloric acid. The organic layer was removed and extracted three times with 10ml of 1-N hydrochloric acid. The aqueous phase was all collected, made alkaline with 20 ml of a 4-N sodium hydroxide solution and extracted twice with 20 ml of dichloromethane. The organic layer was dried over anhydrous sodium sulfate, and the solvent was then distilled off to obtain 1.11 g of a plate yellow solid in a yield of 84.7%.

One gram of the above-obtained pale yellow solid was heat-recrystallized from 8ml of hexane to obtain 0.918g of N-tert-butyltetrahydroisoquinolinecarboxamide as a white crystal in a yield of 91.8%.

Optical rotation: [α]^{D}₂₀ = -111.57 (c=1.0, MeOH) ¹HNMR(CDC₃) :7.2-7.1,7.1-7.0(m,5H,arom and NHCO), 3.99, (s,2H,NH-CH₂-arom),3.43(dd,1H,J=10.7Hz,5.1Hz, NH-CH-CO), 3.21(dd,1H,J=16.5Hz,4.9Hz,CH-CH₂- arom),
2.79(dd,1H,J=16.5Hz,10.7Hz,CH-CH₂-arom), 1.65(s, 1H, NH-CH₂-arom),
1.37(s, 9H, t-Bu),
13CNMR(CD13):172.2, 135.8, 134.5, 129.3, 126.5, 126.1, 125.5, 57.1, 50.6, 47.8, 31.1, 28.7

### Synthesis Example 2

Two grams (11.3 mmols) of tetrahydroisoquinolinecarboxylic acid and a solution of 1.33g (4.48 mmols) triphosgene in 20 ml of tetrahydrofuran were heated and stirred at 60°C for 3 hours. The hot reaction solution was filtered, and the filtrate was cooled to 0°C for 4 hours to obtain 781 mg of NCA as pale yellow crystals (34.1% yield).
IR : 1635, 1459, 1403, 1318, 744 cm⁻¹
1HNMR (DMSO-d6):7.3-7.2(m,4H,arom),4.78(d,1H,J=16.7Hz,arom-CH₂-N),4.46(d,1H,J=16.7Hz,arom-CH₂-N), 4.62(dd,1H, J=5.6Hz, 10.8Hz,CH-N),3.3-3.1(m,2H,CO-CH-CH₂)
13CNMR(DMSO-d6) :169.8,150.7(N-CO-O), 131.2,130.4,129.4,127.0,126.9,126.7,54.3,41.9,28.9

### Synthesis Example 3

Two grams (8.61 mmols) of N-tert-butyltetrahydroisoquinolinecarboxamide and a solution of 0.20g (98.4 mmols) of 5-% Ru/C in 20 ml of 2-propanol were stirred in an autoclave for 20 hours first at room temperature and a hydrogen pressure of 30 atm and then at 100°C. Subsequently, the reaction mixture was cooled, and the catalyst was separated by filtration. The filtrate was distilled off under reduced pressure, and the residue was crystallized from hexane to obtain 1.07 g of N-tert-butyldecahydro(4aS,8aS)-isoquinoline-3(S)-carboxamide as primary crystals (52.1% yield) and 0.42 g of secondary crystals (20.7% yield).
Melting point: 116 - 117°C
Optical rotation: [α]^{D}₂₀ = -72.7 (c=0.5, methanol)
1HNMR(CDC13):6.54(bs,1H,CO-NH),3.1-3.0(m,1H,CH-NH),2.9-2.7(m,2H,CH₂-NH), 1.9-1.2 (m, 13H, cyclohexane ring, CO-CH-CH₂, and CH-NH),
   1.37 (s, 9H, t-Bu)
13CNMR(CDC13):173.5(CO),61.7,51.7,50.4,35.5,34.4,31.8,29. 6,28.8,26.4,9,20.7

### Synthesis Example 4

Phosgene gas (1.17kg) was introduced into a mixture of dichloromethane (9.8L) and tetrahydrofuran (1.7L) at -5°C. To this solution, tetrahydroisoquinoline-3(S)-carboxylic acid (1.05kg) in dichloromethane (4.5L) and tetrahydrofuran (0.8L) was added. After the reaction mixture had been stirred at 45°C for 20 hours, the solvent was distilled to dryness. To the residue dichloromethane (17.8L) was added and the solvent was distilled off again. The resulting slurry, which was cooled to 0°C, was added to a solution of 1.30 kg of tert-butylamine in 6.1 L of dichloromethane at under 5°C for 1 hour. After 1 hour, 14.0 L of water was added, and the organic layer was separated. The organic layer was washed with 3.5 L of water and was reversely extracted two times with 7.0 L of 1-N hydrochloric acid. The aqueous layer was treated with active carbon at 75°C and was filtered. The filtrate was neutralized with 1.4L of 29% sodium hydroxide solution. After cooling at 0°C, the precipitated crystals were separated and dried to give 1.00 kg of N-tert-butyltetrahydroisoquinolinecarboxamide as colourless crystals, a yield of 72.5%.

### Synthesis Example 5

A solution of tetrahydroisoquinoline-3(S)-carboxylic acid (10g) and triphosgene(7.4g) in 100 ml of tetrahydrofuran was stirred for 5 hours at 55°C, for 4 hours at 60° and for 1 hour at 65°C. Fifty ml of solvent was distilled off and fifty ml of heptane was added. The precipitated crystals were filtered, washed with 10 ml of heptane, and dried under reduced pressure overnight to give 9.34 gram of NCA (81.5% yield).

### Synthesis Example 6

To a solution of N-tert-butyltetrahydroisoquinolinecarboxamide(5g) and 5% Ru/C (0.50g) in 33ml of 2-propanol in an autoclave, 30 atm of hydrogen gas was introduced at room temperature. After being stirred for 16 hours at 100°C, the reaction mixture was cooled to room temperature and the catalyst was separated. To the reaction mixture, 15ml of heptane was added. After cooling at 0°C, crystals were precipitated. They were filtered off and dried to give 3.14 gram of white crystals of N-tert-butyldecahydro(4aS,8aS)isoquinoline-3(S)-carboxamide(61.2% yield).

## Claims

1. A process for producing a tetrahydroisoquinoline-3(S)carboxamide derivative represented by formula (3), which comprises reacting tetrahydroisoquinoline-3(S)-carboxylic acid represented by formula (1) with phosgene, phosgene dimer or triphosgene to form N-carboxy anhydride (NCA) represented by formula (2), and then reacting this NCA (without its being isolated or purified) with tert-butylamine: 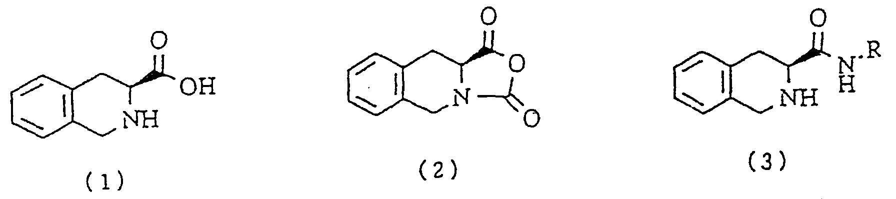 wherein R represents a tert-butyl group; thereby to produce the optically active amide.

2. A process for producing a decahydro (4aS,8aS) isoquinoline-3 (S)-carboxamide derivative represented by formula 4: 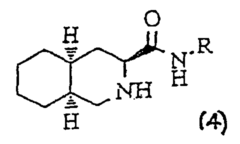 (wherein R represents a tert-butyl group)
which comprises producing a tetrahydroisoquinoline-3(S)-carboxamide derivative of formula (3) by the process of claim 1, and reducing this derivative in the presence of a metal catalyst.

3. The process of claim 2, wherein the metal catalyst is Ru.

4. Tetrahydroisoquinoline-3(S)-carboxylic acid N-carboxy anhydride represented by formula (2).

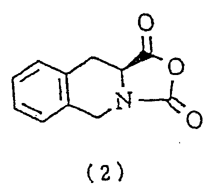

## Patentansprüche

1. Verfahren zur Herstellung eines Tetrahydroisochinolin-3(S)carbonsäureamid-Derivats, das durch Formel (3) dargestellt ist, welches die Umsetzung von Tetrahydroisochinolin-3(S)-carbonsäure, dargestellt durch Formel (1), mit Phosgen, dem Dimeren von Phosgen oder Triphosgen unter Bildung des N-Carbonsäureanhydrids (NCA) dargestellt durch Formel (2), und anschließende Umsetzung dieses NCA (ohne Isolierung oder Reinigung) mit tert-Butylamin umfaßt: 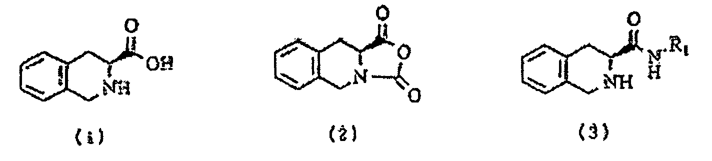 wobei R eine tert-Butylgruppe darstellt, wobei das optisch aktive Amid erhalten wird.

2. Verfahren zur Herstellung eines Decahydro(4aS,8aS)isochinolin-3(S)-carbonsäureamid-Derivats, dargestellt durch Formel (4) 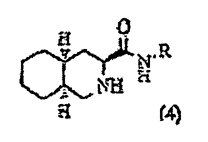 (worin R eine tert-Butylgruppe bedeutet) welches die Herstellung eines Tetrahydroisochinolin-3(S)-carbonsäureamid-Derivats der Formel (3) mit Hilfe des Verfahrens gemäß Anspruch 1 und die Reduktion dieses Derivats in Gegenwart eines Metallkatalysators umfaßt.

3. Verfahren nach Anspruch 2, wobei der Metallkatalysator Ru ist.

4. Tetrahydroisochinolin-3(S)-carbonsäure-N-carbonsäureanhydrid, dargestellt durch Formel (2)

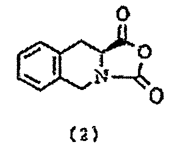

## Revendications

1. Procédé pour produire un dérivé de tétrahydroisoquinoléine-3(S)carboxamide représenté par la formule (3), qui comprend la réaction d'acide tétrahydroisoquinoléine-3(S)-carboxylique représenté par la formule (1) avec du phosgène, du phosgène dimère ou du triphosgène, pour former un N-carboxyanhydride (NCA) représenté par la formule (2), et ensuite la réaction de ce NCA (sans qu'il soit isolé ou purifié) avec la tert-butylamine : 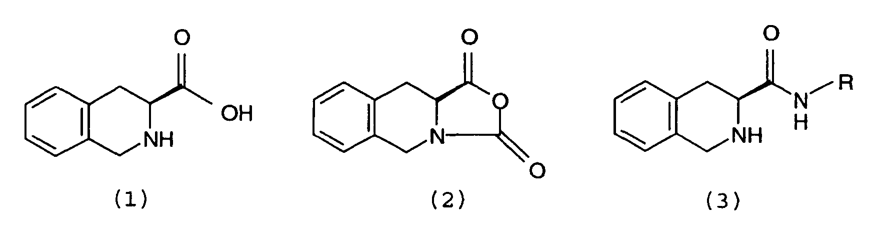 où R représente un groupe tert-butyle ; pour produire l'amide optiquement actif.

2. Procédé pour produire un dérivé de décahydro(4aS,8aS)isoquinoléine-3 (S)-carboxamide représenté par la formule 4 : 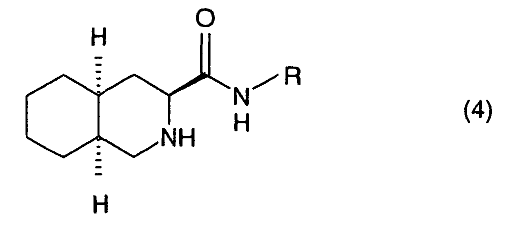 (dans laquelle R représente un groupe tert-butyle) qui comprend la production d'un dérivé de tétrahydroisoquinoléine-3(S)-carboxamide de formule(3) par le procédé de la revendication 1, et la réduction de ce dérivé en présence d'un catalyseur métallique.

3. Procédé selon la revendication 2, dans lequel le catalyseur métallique est Ru.

4. N-carboxyanhydride d'acide tétrahydroisoquinoléine-3(S)-carboxylique représenté par la formule (2) :
